# EUROPEAN PATENT APPLICATION

(11) **EP 2 153 815 A1**
(43) Date of publication of application: **17.02.2010**
(21) Application number: 08013996.7
(22) Date of filing: 05.08.2008
(51) Int. Cl.: A61K 8/42

(54) **Use of urea containing compositions**

(71) Applicant: ISDIN S.A., 08006 Barcelona (ES)
(72) Inventor: Trullas Cabanas, Carlos Ramon, 08232 Viladecavalls (ES); Krutmann, Jean Prof. Dr., 40225 Düsseldorf (DE)
(74) Representative: Brosch, Oliver

(57) **Abstract**

The present invention relates to the use of a composition comprising urea for increasing the expression of at least one gene associated with the collagen synthesis within the dermis of the skin.
The urea composition has been used for preventing/treating damages of the skin such as signs of aging, wrinkles, damages associated with UV-radiation, wounds, skin diseases.

## Description

The present invention relates to the use of compositions comprising urea for increasing the expression of genes, preferably col1A1/col1A2 in the dermis of the skin, associated with the synthesis of collagen type I and for increasing the amount of collagen type I within the skin. Furthermore the invention relates to the use of said compositions for the prevention of damages of the skin associated with and/or facilitated by a reduced gene expression or damages caused by exposure to UV-irradiation and/or other external factors.

The skin of mammals, especially human beings is a complex organ being subdivided into many different layers. Each layer serves a specific purpose and thus expresses specific markers and comprises specific structures.

The outer layers of the skin collectively constitute the epidermis. It is the thinnest arrangement of layers of the skin and has the important function to moisturize and protect. More specifically, the epidermis protects the body from desiccation and the invasion of noxious substances, UV light, virus and bacteria. The epidermis is subdivided (from superficial to deep (bordering the dermis)) in the stratum corneum, stratum lucidum (only present at palms and soles), stratum granulosum, stratum spinosum and stratum germinativum (also known as stratum basale).

The layer underneath the epidermis is known as the dermis and is tightly connected to the epidermis by a basement membrane.

The dermis comprises of connective tissue and contains numerous nerve endings that provide the sense of touch and heat. The dermis is also the layer of the skin, wherein hair follicles, sweat glands, sebaceous glands, apocrine and blood vessels are located. The latter also provide nutrients for the epidermis which is not directly linked to the blood system.

The dermal layers neighboring the epidemis are called papillary region due to myriads of papillae which extend into the epidermis and thereby strengthening the connection between these two layers of the skin.

The deeper layers of the dermis are called reticular region. This tissue is composed of dense irregular connective tissue being composed of fibroblast cells and their extracellular matrix. It was named because of the dense concentration of collagenous, elastic, and reticular fibers that weave throughout it. These protein fibers, largely consisting of collagen, give the dermis strength, extensibility, and elasticity.

Collagens are ubiquitously within the human body. They are present in all human connective tissues, such as dermis, bones, tendons, and ligaments, and are responsible for the structural integrity of all of our internal organs. Therefore, because of its wide distribution throughout our bodies, it represents one of the most abundant naturally occurring proteins on earth.

There are several different types of collagens, each having a specific expression pattern. The (major) collagen which is present in the skin is collagen type I. This fibrillar form of collagen represents over 90 percent of the total collagen within the human body and is composed of three very long protein chains. Each protein chain is referred to as an "alpha" chain. Two of the alpha chains are identical and are called alpha-1(I) chains, whereas the third chain is slightly different and is called alpha-2(I). The three chains are wrapped around each other to form a triple helical structure called a collagen monomer. This configuration imparts tremendous strength to the protein.

Type I Collagen is encoded by the genes col1A1 and col1A2. The first gene encodes the pro-alpha1(I)-chains, the latter the pro-alpha2(I)-chain. These chains then form procollagen which is further processed outside the cell.

Once the collagen is processed, they arrange themselves into long, thin fibrils that cross-link to one another in the spaces around cells. The cross-links results in the formation of very strong mature type I collagen fibers.

Collagen is degraded by collagenase. Another key enzyme for the degradation of collagen is matrix metalloproteinase

Thus the collagen fibers play a pivotal role for the integrity and the functioning of the skin.

A strong network of collagen fibers protects the skin against damages. It is thus essential e.g. for the barrier-function of the skin against many natural and synthetic substances and also for protecting the body against physical irritations.

This is illustrated e.g. by genetic disorders such as the Ehlers-Danlos syndrome or Osteogenesis imperfecta, in which genes associated with the collagen metabolism are defect.

The skin of patients suffering from the Ehlers-Danlos-classica-syndrom tends to be soft, highly elastic and may tear, bruise or scar easily and may heal slowly. The skin of patients suffering from Ehlers-Danlos-arthrochalasis-syndrome may be stretchy and fragile.

With playing such an important role for the structural integrity of the skin, an intact collagen metabolism is essential for wound healing. If all skin layers (not only the epidermal layers) are injured, new connective tissue must fill in the wound space. Such tissue, named granulation tissue, is formed by deposition of extracellular matrix components such as collagen by fibroblasts which migrate into the wound space.

The synthesis of collagen is even more important when large areas of the skin are injured such as by burns.

As further outlined below, the status of the collagen network is furthermore tightly associated with the aging processes within the skin.

With the progressing aging of the skin, some or all of the following features may appear:
Fine wrinkles, thin and transparent skin, loss of underlying fat which leads to hollowed cheeks and eye sockets as well as noticeable loss of firmness of the skin on the hands and neck, shrinking away of bones from the skin due to bone loss causing sagging skin, dry skin, inability to sweat sufficiently, graying hair, thinning of the nail plate, skin atrophy appearance and/or depth of lines and/or wrinkles, including fine lines, skin discoloration, including dark eye circles, skin sagging, skin fatigue and/or stress, e.g. skin breakout, skin flakiness, cellular aging, loss of skin tone, elasticity and/or luster, loss of skin firmness, poor skin texture, loss of skin elasticity and/or resilences.

Processes associated with aging are caused by intrinsic as well as extrinsic factors.

Intrinsic aging, also known as the natural aging process, is a continuous process that normally begins in the mid-20s of human beings. Within the skin, collagen production slows, and the elastin-structures becomes less flexible. Dead skin cells do not shed as quickly and turnover of new skin cells may decrease slightly. While these changes usually begin in the 20s, the signs of intrinsic aging are typically not visible for decades.

An important intrinsic factor facilitating signs of aging on the skin are hormonal changes e.g. during menopause.

A number of extrinsic, or external, factors often act together with the normal aging process to age the skin prematurely. Most premature aging is caused by sun exposure. Other external factors that age the skin prematurely are repetitive facial expressions, gravity, sleeping positions, temperature, humidity, environmental pollution, irritants (detergents, acids, bases, oxidizing agents, reducing agents, concentrated solvents, toxic gases or fumes), mechanical stresses (friction, impacts, abrasion, tearing of the surface, projection of dusts or particles, shaving or hair removal), thermal or climatic imbalances (cold, dryness or radiation), xenobiotics (undesirable microorganisms, allergens) and smoking.

Also, e.g. chronobiological stress accelerates aging thereby also affecting the skin.

Sun exposure leads to photoaging. The skin loses the ability to repair itself, and the damage accumulates. Repeated ultraviolet (UV) exposure breaks down collagen fibers and impairs the synthesis of new collagen. The sun also attacks elastin. Sun-weakened skin ceases to be resilent much earlier than skin protected against UV rays. Skin also becomes loose, wrinkled, and leathery much earlier because of unprotected exposure to sunlight.

Some of the signs of aging mentioned above are in particularly prominent in photoaging. The photoaged skin can often be the thicker than normal (acanthotic) and then atrophies over time. See also N. A. Fenske and C. W. Lober, "Structural and functional changes of normal aging skin," J. Am. Acad. Dermatol., 15:571-585 (1986).

Thus photoaging is in particular characterized clinically by coarseness, wrinkles, mottled pigmentation, sallowness, laxity, telangiectasia, lentigines, purpura and relative ease of bruising, atrophy, depigmented areas, eventually premalignant, and ultimately malignant neoplasms.

In each case however, a prominent feature associated with aging on the molecular level is a decrease in the collagen-content. This is caused by a decrease in collagen synthesis as well as an increase in collagen degradation.

Enormous efforts have been undertaken in the past to slow down aging or at least to render the effects of these processes (the signs of aging) less visible.

Numerous efforts have been based on scientific reasoning, other less so. Numerous efforts have been directed on combating signs of aging which already started to appear on the skin. Thus, e.g. multitudinous cremes, lotions and the like have been developed for moisturizing the skin combating increasing dryness, wrinkles etc.

Also a large number of approaches have been tested aiming at increasing the collagen content of the dermis.

An increase in the collagen content has both a direct effect on the structural integrity of the dermis as well as an indirect effect. This is because when the collagen metabolism is activated by an increase in collagen synthesis, optionally resulting in an increase of other dermal matrix components.

Products obtained by mixing collagen with a composition for external application to the skin have been developed. However, due to its macromolecular structure, collagen is not able to penetrate the intact skin in sufficient quantities.

Another approach for increasing the collagen content was to supplement the intrinsic collagen with extrinsic collagen. Thus various regimens for injecting collagen have been developed. Alternatively other substances are being injected which have an impact on the collagen content of the dermis (For a review of these approaches including a discussion of the advantages and disadvantages see e.g. Epply BL and Dadvand B (2006) "Injectable soft-tissue fillers: clinical overview" Plast. Reconstr. Surg. 118: 98e-106e and Krauss MC (1999) "Recent advances in soft tissue augmentation" Semin. Cutan. Med. Surg. 18: 119-28). Despite requiring a physician and being of only limited convenience, these injections have become widespread and are gladly accepted by significant numbers of people. Thus, indicating a demand for increasing the collagen level in the skin.

Another approach has been to develop substances for reducing the collagenase activity. Examples thereof are being described e.g. in W007094533 ("Composition for inhibiting collagen degradation and promoting collagen synthesis comprising emodin") or W007075016 ("Collagenase inhibitor containing poly-gamma-glutamic acid vitamin C complex and use thereof").

Furthermore numerous undertakings were aimed at identifying substances for stimulating collagen synthesis. Well known substances are vitamin C, retinol, certain phytokines and coenzyme Q10 among others.

It is also known that amino acids that are the building blocks of proteins also promote collagen synthesis

It has been reported from studies using fibroblasts that glutamine promoted collagen synthesis (G. Bellon et al. (1995) Biochimica Biophysica Acta, 1268, 311-323).

Likewise the following compounds/compositions have been described:
An amino acid composition as disclosed in EP1582208A3 ("An amino acid compositions for promoting collagen synthesis")
Certain fusion peptides grafted with TAT-peptide to human type-I collagen derived peptide as disclosed in WO03078470A1 ("Fusion peptide grafted with TAT-peptide to human type-I collagen derived peptide, its preparation method and cosmetic composition for anti-aging comprising the same")
Other substances which can promote the synthesis of collagen are TGF (see Cardinale G et al. (1974) Adv. Enzymol 41: 425), a protein originated from animal placenta (JP08231370A2 " Skin cosmetic"), betulinic acid (JP08208424A2 "Cosmetic composition containing betulinic acid")
Similarly, compounds have been developed for reducing UV-induced inhibition of collagen synthesis thereby stimulating collagen synthesis (see e.g. the compounds as disclosed in W09951220 "Methods and compositions for reducing UV-induced inhibition of collagen synthesis in human skin").

Additionally, also some studies have indicated a role of various substances in promoting collagen synthesis.

Examples of such substances or combinations are like a composition comprising a bertholletia extract as disclosed in EP0809484 ("Cosmetic or pharmaceutical, particularly dermatological, composition containing a bertholletia extract"); certain bisphosphonates, e.g. pamindronate, as disclosed in EP1566177 ("The use of bisphosphonates for treating skin by stimulating collagen synthesis"); an AIMP1-fragment as disclosed in W006083087 ("Method for stimulating collagen synthesis and/or KGF expression"); a composition comprising emodin as disclosed in W007094533 ("Composition for inhibiting collagen degradation and promoting collagen synthesis comprising emodin"); compounds represended by Chemical Formula 1 of WO05/037270 ("Composition for promoting synthesis of collagen, and composition for external preparation for skin comprising the same"), in particular is xanthotoxol, 8-hydroxypsoralen; 8-hydroxybergapten, prangenidin, i.e., 5 Benzofuranacrylic acid, 6, 7-dihydroxy-4-(3-methyi-2-butenyl)-deita- lactone.

The object of the present invention is to prevent and/or to treat damages whose occurrence and/or severity are dependent on the level of production of collagen in the skin.

Surprisingly, it was found that topical application of urea results in an increased expression of genes involved in collagen de novo synthesis within the skin, namely within the dermis. Accordingly, it was found that the application of urea leads to an increase in the collagen content in the treated skin.

Accordingly, the present invention relates to the use of a composition comprising urea for increasing the expression of at least one gene associated with the collagen synthesis within the dermis of the skin.

The term "collagen" as used according to the present invention refers to collagen type I of mammals, preferable human beings.

The term "dermis" as used according to the present invention means the dermis of mammals' skin, especially of humans' skin.

The term "composition" as used according to the present invention comprises but is not limited to cosmetic and pharmaceutical compositions.

The person skilled in the art will be able to specifically adapt the inventively used composition according to the intended application/administration to the skin, especially the dermis. Exemplary formulations of application/administration are outlined further below.

The person skilled in the art will be able to specifically adapt the composition according to the invention with respect to any regions of the human skin to which said composition should be administered.

Thus, the person skilled in the art will be able to select and/or avoid auxiliary substances which might be suitable or unsuitable for use in the different regions of the skin, e.g. eyes, face, neck, arms or legs etc.

Such a selection of components for an inventively used composition has also to take into account that such component does not infer with the effect of urea.

Subject to these provisions and depending on the specific requirements of the application, the inventively used compositions may contain one or more of the following of compounds: gelling agents, oils, waxes, thickening agents, hydrophilic or hydrophobic polymers, moisturizers, emulsifying agents, emollients, fatty acids, organic solvents, stabilizers, sequestering agents, acidifying or basifying agents, surfactants, film formers, biological additives to enhance performance and/or consumer appeal such as amino acids, proteins, vanilla, aloe extract or bioflavinoids, buffering agents, chelating agents such as ethylenediaminetetra-acetic acid (EDTA) or oxalic acid, colorants, dyes, propellants, antifoaming agents, wetting agents, vitamins, emulsion stabilizers, pH adjusters, thickening agents, fragrances, fillers, preservatives, opacifying agents, water and/or alcohols. The aforementioned auxiliary agents for the inventively used compositions are used in the usual amounts known by those skilled in the art.

As oils for the inventively used compositions oils from animal or vegetable sources or synthetic are preferably used. As oils can be used at least one oil selected from the group comprising mineral oil, liquid petrolatum, liquid paraffin, volatile and non-volatile silicone oils, isoparaffins, polyalphaolefins, fluorated and perfluorated oils. Examples of further suitable oils - depending on the compounds of the composition - comprise hydrocarbon-based oils of animal origin such as squalene, perhydrosqualene; hydrocarbon-based plant oils such as liquid triglycerides of fatty acids of 4 to 10 carbon atoms, for instance, heptanoic or octanoic acid triglycerides, or oils such as olive oil, macademia nut oil, sunflower oil, sunflower seed oil, corn oil, soybean oil, grapeseed oil, castor oil, avocado oil, caprylic/capric acid triglycerides, jojoba oil; linear or branched hydrocarbons of mineral or synthetic origin such as liquid paraffins and derivatives thereof, petroleum jelly; synthetic esters and ethers, in particular esters of fatty alcohols, like; for example, octyldodecyl myristate, glyceryl octanoate, ethylhexyl isononanoate,isopropyl myristate, 2-ethylhexyl palmitate, 2-octyldodecyl stearate, isostearyl isostearate; hydroxylated esters such as isostearyl lactate, octyl hydroxystearate, octyldodecyl hydroxystearate, heptanoates, octanoates and decanoates of fatty alcohols; polyol esters such as propylene glycol dioctanoate, neopentyl glycol diheptanoate, diethylene glycol diisononanoate, and pentaerythritol esters; fatty alcohols containing from 12 to 26 carbon atoms such as octyldodecanol, 2-butyloctanol, 2-hexyldecanol, 2-undecylpentadecanol, oleyl alcohol; partially hydrocarbon-based fluoro oils and/or fluorosilicone oils; silicone oils such as volatile or non-volatile, linear or cyclic polymethylsiloxanes (PDMS) that are liquid or semisolid at room temperature such as cyclomethicones and dimethicones, optionally comprising a phenyl group, for instance phenyl trimethicones, siloxanes, and mixtures thereof.

The oil phase of the inventively used compositions may comprise one or more waxes, gums, or mixtures thereof. The waxes include hydrocarbon-based waxes, fluoro waxes and/or silicone waxes and can be of plant, mineral, animal and/or synthetic origin.

The gums are e.g. high molecular weight PDMSs, cellulose gums or polysaccharides, and the semi-solid materials are generally hydrocarbon-based compounds, such as, but not limited to, lanolins and derivatives thereof, or alternatively PDMSs.

The term "moisturizer" as used according to the present invention means a compound that acts on the barrier function, in order to maintain the moisturization of the stratum corneum, or an occlusive compound. Mention may be made of ceramides, sphingoid-based compounds, lecithins, lycosphingolipids, phospholipids, cholesterol and derivatives thereof, phytosterols (stigmasterol, β-sitosterol or campesterol), essential fatty acids, 1,2-diacylglycerol, 4-chromanone, pentacyclic triterpenes such as ursolic acid, liquid petroleum jelly and lanolin; or a compound that directly increases the water content of the stratum corneum, such as threalose and derivatives thereof, hyaluronic acid and derivatives thereof, glycerol, pentanediol, sodium pidolate, serine, xylitol, sodium lactate, polyglyceryl acrylate, ectoin and derivatives thereof, chitosan, oligosaccharides and polysaccharides, cyclic carbonates, N-lauroylpyrrolidonecarboxylic acid and N-α-benzoyl-L-arginine; or a compound that activates the sebaceous glands, such as DHEA, 7-oxide and/or 17-alkyl derivatives thereof, sapogenins and vitamin D and derivatives thereof.

As stabilizers for the inventively used compositions preferably non-ionic, anionic, cationic and amphiphilic tensides can be used, which are preferably selected from the group comprising polyethylenglycol (PEG) and derivatives thereof, tweens, tritons, spans, polygycerines, polyalkyl glycerides, alkyl sulfonates, aryl sulfonates, alkyl phosphates, derivatives of alkyl-betaine and phosphatidylglycerole.

Emulsifiers are preferably used in certain formulations of the inventively used compositions in amounts effective to provide uniform blending of ingredients of the composition. Useful emulsifiers include anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium acetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and acetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate. Preferred emulsifiers are glycerol stearate, PEG-40-stearate, sodium carbomer, acrylate/C10-C30-alkyl acrylate crosspolymer.

Surfactants can be used in certain formulations of the inventively used compositions. Suitable surfactants are for example those surfactants generally grouped as cleansing agents, emulsifying agents, foam boosters, hydrotropes, solubilizing agents, suspending agents and non-surfactants, which facilitate the dispersion of solids in liquids. Examples of the surfactants include sorbitan sesquioleate, polysorbate 60, glyceryl stearate, lipophilic glyceryl stearate, sorbitan oleate, sorbitan stearate, DEA-cetyl phosphate, sorbitan stearate/sucrose cocoate, glyceryl stearate/polyethylene glycol- 100 stearate, ceteareth-6 olivate, arachidyl alcohol/behenyl alcohol/arachidyl glucoside, polypropylene glycol- 26-buteth-26/polyethylene glycol-40, and hydrogenated castor oil. Glycerin, sorbitol, propylene glycol, butylene glycol, hexylene glycol, diglycerin, betaine, glycereth-26, or methylgluceth-20 can be used as humectants. Cetyl alcohol, stearyl alcohol, octyl dodecanol, and isostearyl alcohol can be used as higher alcohols.

Xanthan Gum, carbomer, magnesium aluminium silicate, cellulose gum, and/or dextrin palmitate can be used as thickeners. Disodium EDTA and tetrasodium EDTA can be used as chelating agents. Blue No. 1, Red No. 40, and Yellow No. 4 and 5 can be used as colorants.

Suitable film formers which are preferably used in the formulations of the inventively used compositions should keep the composition smooth and even and are preferably, without limitation, at least one substance selected from the group comprising acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (vinyl methylether/maleic acid anhydride copolymer); PVP (polyvinylpyrrolidone); maleic acid anhydride polymer, vinylpyrrolidon/hexadecene copolymer; acrylate copolymer and the like.

The person skilled in the art will be able to adjust the pH-value of the inventively used composition to a suitable pH-value for the particular application.

pH adjusters may also be used in certain formulations of the inventively used compositions. These pH adjusters preferably comprise, but are not limited to ammonium hydroxide, triethanolamine or citric acid.

As further thickening agents for the inventively used compositions can be preferably used candelilla, carnauba, and microcrystalline waxes, crosslinked acrylic-acid polymers, carbomer, methylhydroxyethylcellulose, hydroxypropylmethylcellulose or hydroxyethylcellulose. and polyethylene thickeners.

Gelling agents, which can be preferably used in the formulations of the inventively used compositions, can be natural or synthetic polymers. Natural polymers are preferably selected from the group comprising Agar-Agar, alginate, pectin, carbomer, carrageenan, casein, dextrine, gelatine, arabic gum, keratine, locust bean gum, xanthan gum and the like. Preferred synthetic polymers, which can be used in the formulations of the inventively used compositions, are selected from the group comprising acylic acid polymers, polyacryl amides and alkylene oxide polymers.

Exemplary fillers include but are not limited to talc, silica, zinc stearate, mica, kaolin, nylon (in particular orgasol) powder, polyethylene powder, Teflon, starch, boron nitride, microspheres of copolymers such as Expancel (Nobel Industrie), Polytrap (Dow Coming), and silicone resin microbeads (Tospearl from Toshiba).

The inventively used composition may also include e.g. a skin penetration enhancer, a skin plumber and/or an optical diffuser.

As skin plumpers a collagen enhancer to the skin can serve. An example of a suitable, and preferred, skin plumper is palmitoyl oligopeptide. Other skin plumpers are collagen and/or glycosaminoglycan (GAG) enhancing agents. The skin plumper can be preferably present in an amount of from about 0.1 wt % to about 20 weight %, based on the total weight of the composition. An optical diffuser comprises particles that change the surface optometrics of skin, resulting in a visual blurring and softening of, for example, lines and wrinkles. Examples of such optical diffusers that can be used in the presently inventively used compositions include, but are not limited to, boron nitride, mica, nylon, polymethylmethacrylate (PMMA), polyurethane powder, sericite, silica, silicone powder, talc, Teflon, titanium dioxide, zinc oxide, or any mixtures thereof. An optical diffuser is preferably present in an amount of from about 0.01 weight % to about 20 weight %, based on the total weight of the composition.

The inventively used composition may also include nutritionally desirable minerals.

Other additives include beneficial agents such as those materials that condition.

Other additives include beneficial agents such as those materials that condition the skin (particularly, the upper layers of the skin in the stratum corneum) and keep it soft by retarding the decrease of its water content and/or protect the skin. Such conditioners and moisturizing agents include, by way of example, pyrrolidine carboxylic acid and amino acids; Other additives comprise cosmetically and/or cosmeceutically active agents. Such additional cosmetical and/or cosmeceutically active agents include, for example, alpha hydroxyacids, alpha ketoacids, polymeric hydroxyacids, moisturizers, collagen, marine extract.

Other agents may also be added, such as antimicrobial agents, to prevent spoilage upon storage, i.e., to inhibit growth of microbes such as yeasts and molds. Exemplary organic antimicrobrial agents are e.g. 2,4,4'-trichloro-2-hydroxy diphenyl ether (triclosan) and benzoic acid.

The compositions may also contain irritation-mitigating additives to minimize or eliminate the possibility of skin irritation or skin damage resulting from the chemical entity to be administered, or other components of the composition. Exemplary irritation-mitigating additives include, for example: α-tocopherol; monoamine oxidase inhibitors, particularly phenyl alcohols such as 2-phenyl-1-ethanol; glycerin; salicylates; ascorbates; ionophores such as monensin; amphiphilic amines; ammonium chloride; N-acetylcysteine; capsaicin; and chloroquine.

The compositions may also contain anti-inflammatory agents. Exemplary anti-inflammatory agents are e.g. acetylsalicylic acid and glycyrrhetinic acid.

The compositions may also contain anti-seborrhoeic agents, vasodilators, inhibitors of melanogenesis, anti-allergenics, antifungals, antiseptics, , analgesics, nitric oxide synthase inhibitors, insect repellents, self-tanning agents, skin penetration enhancers, skin cooling agents, chelating agents, colorants including dyes, lakes and pigments that may be untreated or chemically surface treated to improve wetability or some other property, pearlescent agents, demulcents, agents for stimulating the synthesis of dermal or epidermal macromolecules and/or for preventing their degradation; agents for stimulating fibroblast proliferation and/or for stimulating keratinocyte differentiation; muscle relaxants; tensioning agents; antipollution agents and/or free-radical scavengers, sunscreens and mixtures thereof, anesthetics, antineoplastics, antivirals, hypopigmenting agents, immune system boosting agents, immune system suppressing agents, insect repellents, lubricants, matting agents, photostabilizing agents, preservatives, skin protectants, skin penetration enhancers, staining agents, sunscreens, viscosity and/or rheology modifiers.

The pigments which may be added to the inventively used compositions comprise inorganic pigments and organic pigments. Suitable inorganic pigments include, but are not limited to, titanium oxide, zirconium oxide and cerium oxide, as well as zinc oxide, iron oxide, chromium oxide and ferric blue. Suitable organic pigments include, but barium, strontium, calcium, and aluminium lakes and carbon black.

Suitable pearlescent agents include mica coated with titanium oxide, with iron oxide, or with natural pigment.

The depigmenting agents that may be incorporated into the composition according to the present invention comprise, for example, the following compounds: kojic acid; ellagic acid; arbutin and derivatives thereof such as those described in U.S. Pat. No. 6,306,376 and U.S. Pat. No. 5,346,693; hydroquinone, without this list being limiting.

The composition according to the invention may comprise dermo-decontracting agents, among which mention may be made in particular of alverine and salts thereof, especially alverine citrate, sapogenins such as diosgenin and natural extracts containing it (such as extracts of wild yam), certain secondary and tertiary carbonyl amines, organic or mineral salts of metals, in particular manganese gluconate, adenosine, and also the hexapeptide argireline R sold by the company Lipotec. Mention may also be made of certain fragrancing compositions with a dermo-decontracting effect.

As far as the invention relates to (a use of) a cosmetic composition, it is further referred to International Cosmetic Ingredient Dictionary and Handbook (ICID), 10th Edition (2004), published by the Cosmetic, Toiletry, and Fragrance Association (CTFA), at pp. 2177-2299, which includes information about suitable cosmetic ingredients and their use and which is herein incorporated by reference in its entirety.

As it will be obvious to the person skilled in the art, the composition may contain many more compounds suitable for the specific requirement. Some further categories of compounds are disclosed herein after.

Depending on the specific constitution of any of the inventively used compositions, the person skilled in the art will be able to select the appropriate steps for preparing said composition.

The particular combination of components in the composition is determined largely by chemical compatibility. The person skilled in the art will be able to easily determine which of the compounds listed above and which alternative compounds known to the person skilled in the art might be used/combined for a specific composition according to the invention.

In a further embodiment, the present invention relates to the use of a composition comprising urea for increasing the expression of col1A1 and/or col1A2 within the dermis of the skin.

As known to the person skilled in the art, the gene col1A1 encodes the protein collagen type I alpha 1, the gene col1A2 encodes the protein collagen type I alpha 2.

Surprisingly it was found that said use increases the expression of said genes considerably.

Therefore, the present invention also relates to the use of a composition comprising urea for increasing the expression of a gene according to the invention by a factor of at least 2, preferably at least 3, more preferably at least 4.

As known to the person skilled in the art, various methods are available to determine the rate of expression of a gene.

Inventively said factor is determined by real time PCR as outlined in the section "Example".

According to the present invention said genes encode the before mentioned protein collagen types.

Hence, the present invention also relates to the use of a composition comprising urea for increasing the amount of collagen, by weight, in the treated skin, by at least 5%, preferably by at least 10%, more preferably by at least 20%, even more preferably by at least 50%, most preferably by at least 100%.

The compositions comprising urea are preferably topically applied onto the skin or administered by injection.

For the preferred topical application, the inventively used composition may be formulated in liquid or in semi-solid form, preferably as liquid, fluid, foam, cream, gel, paste, balsam, spray, ointment, lotion, conditioner, tonic, milk, mousse, emulsion, serum, oil, stick, shampoo, jelly, suspension, dispersion, lacquer, paint, elixir, drop, aerosol, day creams or lotions, night creams or lotions, salves, sunscreen creams, fluid lotions, oils, water-in-oil, oil-in-water, water-oil-water triple emulsions, masks, body milks, makeup, artificial tanning compositions, depilatories, emulsifiers, patches, or a solid which is poured or cast as a stick or a dish, microemulsions, aerosol, powder, soap, surfactant-containing cleansing powder foundation, emulsion foundation, wax foundation, softening lotion, nutrient lotion, nutrient cream, massage cream, bath compositions, aftershave gels or lotions, cleansing cream, cleansing foam, cleansing water or packs, composition for countering insect stings or bits, pain-relieving compositions or compositions for treating certain skin diseases or skin disorders. It is also contemplated that topical compositions of the present invention can be incorporated into delivery systems such as liposomes, nano particles and topical patches, tapes, and sprays. It may also be formulated in a makeup product for facial skin, the body or the lips, such as a foundation, a tinted cream, a makeup rouge, an eyeshadow, a loose or compact powder, a concealer stick, a cover stick, a lipstick or a lipcare product.

The compositions containing urea can also be injected into the skin that should be treated.

For such an injection, the composition can be provided in the form of aqueous or oily lotions or in the form of serums.

Preferably, the pH-value of the inventively used composition will be adjusted using a suitable puffering agent. Preferably the pH-value of the inventively used composition will be in the range of 3,5 - 9,0 for topical application.

For injection, the preferred pH-value of the inventively used composition will be in the range of 3,0 - 9,0.

For a successful treatment of the skin the compositions comprising urea is applied onto the skin at least once a day for at least 2 weeks topically and optionally longer until the expression of any of said genes is increased by a factor of at least 2, preferably at least 3, more preferably at least 4 and/or the amount of collagen in the treated region is increased by an amount of collagen, by weight, in the treated skin, preferably by at least 5%, preferably by at least 10%, more preferably by at least 20%, even more preferably by at least 50%, most preferably by at least 100%.

A successful treatment of the skin can also be achieved by injections, preferably by, optionally repeated, injections of a depot.

In total the period of application and/or administration can continue for several weeks or even several months or even several years either continuously or in repeated phases of application.

In general the method of delivery of the active agent may vary, but necessarily involves application of the composition with urea to an area of skin prone to or affected by a damage.

Preferably the compositions comprising urea comprise from 1 to 30% by weight, preferable from 5 to 25% by weight, based on the total amount of the composition, of urea.

The use of the compositions according to the present invention allows to prevent and/or to treat damages of skin.

Therefore the present invention also relates to the use of the composition comprising urea for preventing and/or treating of damages associated with and/or facilitated by a reduced level of collagen in the skin and/or a reduced expression of genes associated with the collagen synthesis in the dermis by increasing the collagen level at least to compensate at least said reduction.

The terms "treating" and "treatment as used according to the present invention refer to weakening the severity and/or elimination of damages according to the invention.

The terms "prevent" or "preventing" as used according to the present invention refer to the avoidance or the slow down of the occurrence of a damage as defined hereinafter.

The term "damages" as used according to the present invention refers to any of the following: signs of aging caused by intrinsic and/or extrinsic factors (a list of signs of aging is included further below). Furthermore, the term includes signs of aging and other damages as set forth thereafter.

According to the present invention the use of a composition comprising urea for preventing and/or treating of damages associated with and/or facilitated by a reduced level of collagen in the skin and/or a reduced expression of genes associated with the collagen synthesis in the dermis can be achieved by treating normal skin or already damaged skin of a mammal, preferable of a human being.

The term "normal skin" as used according to the present invention refers to healthy skin of a 25 year-old male or female respectively taking into account the sex, ethnic background of said male or female as well as the part of the body.

In a still further embodiment, the present invention relates to a use as described above for preventing and/or treating signs of aging.

Signs of aging may include e.g. fine wrinkles, thin and transparent skin, loss of underlying fat which leads to hollowed cheeks and eye sockets as well as noticeable loss of firmness on the hands and neck, shrinking away of bones from the skin due to bone loss causing sagging skin, dry skin, inability to sweat sufficiently, graying hair, thinning of the nail plate, skin atrophy appearance and/or depth of lines and/or wrinkles, including fine lines, skin discoloration, including dark eye circles, skin sagging, skin fatigue and/or stress, e.g. skin breakout, skin flakiness, cellular aging, loss of skin tone, elasticity and/or luster, loss of skin firmness, poor skin texture, loss of skin elasticity and/or resilences.

The composition used according to the invention might be applied to the complete skin surface or at least significant parts thereof. The application might also be limited to specific regions of the body which are particularly vulnerable for factors triggering aging processes. Regions which might be very vulnerable to photoaging are those regions that are typically exposed to sunlight such as the face, ears, bald areas of the scalp, neck, décolleté, lips, forearms, wrists, hands and feet.

Alternatively, the administration might be limited to regions of the skin which are particularly likely to exhibit signs of aging.

Therefore the compositions containing urea are especially useful to prevent and/or treat signs of aging like wrinkles and/or loss of elasticity and/or dischronia, preferable melasma.

For such treatment is preferred a continuous treatment, preferably by adjusting the amount of urea according to an increased need for compensation of the amount of collagen.

According to the present invention the compositions with urea could be used to prevent and/or to treat damages caused by exposure to UV-radiation.

Some of the signs of aging mentioned above are in particularly prominent in photoaging. The photoaged skin can often be the thicker than normal (acanthotic) and then atrophies over time. See also N. A. Fenske and C. W. Lober, "Structural and functional changes of normal aging skin," J. Am. Acad. Dermatol., 15:571-585 (1986).

Thus photoaging is in particular characterized clinically by coarseness, wrinkles, mottled pigmentation, sallowness, laxity, telangiectasia, lentigines, purpura and relative ease of bruising, atrophy, depigmented areas, eventually premalignant, and ultimately malignant neoplasms.

Photoaging commonly occurs in skin that is habitually exposed to sunlight such as the regions of the skins mentioned before.

These regions are also typical regions for the administration of UV-filter-containing compositions. Thus, the compositions used according to the invention might be specifically adapted to the needs of these regions and can optionally contain further compounds which also prevent and/or treat signs of aging.

Thus, in a still further embodiment, the present invention relates to the use of a composition comprising urea adjusted as a cosmetic composition and preferable comprises optionally at least one compound selected from the group comprising UV-filters, vitamins, coenzymes, retinoids, phytokines and antioxidative agents.

At least some of such compounds are known to stimulate the macromolecules of the dermis or prevent their degradation influencing the synthesis of collagen, such as extracts of Centella asiatica; asiaticosides and derivatives; ascorbic acid or vitamin C and its derivatives, such as ascorbyl glucoside (marketed by Hayashibara); synthetic peptides, such as iamine, the palmitoyl of glycine-histidine-lysine tripeptide, marketed under the name "Biopeptide CL" by Sederma; peptides extracted from plants, such as the soybean hydrolysate marketed by Coletica under the trademark Phytokine®; extracts of soya fibers, such as that marketed under the name "Raffermine" by Silab; plant hormones, such as auxins and lignans; the palmitoyl of lysine-threonine-threonine-lysine-serine pentapeptide marketed in particular under the name "Matrixyl" by Sederma; dimethylaminoethanol; extracts of Bupleurum chinensis rhizome, such as those marketed under the names "Pleurimincyl" or "Lipocare" by Sederma; acylated with a hydrolysates of wheat protein, in particular acylated with a palmitoyl group, such as that marketed under the name "Lipacid PVB" by Seppic; creatine; coenzyme Q10; retinol; dipalmitoyl hydroxyproline, in particular marketed by Seppic under the name "Sepilift DPHP", or extracts of red clover (Trifolium pretense) comprising isoflavones; or the synthesis of elastin, such as the extract of Saccharomyces cerivisiae marketed by LSN under the trademark Cytovitin®; and the extract of the alga Macrocystis pyrifera marketed by Secma under the trademark Kelpadelie®; or the synthesis of glycosaminoglycans, such as the product of fermentation of milk by Lactobacillus vulgaris marketed by Brooks under the trademark Biomin yogourth®; the extract of the brown alga Padina pavonica marketed by Alban Müller under the trademark HSP3®; and the extract of Saccharomyces cerevisiae available in particular from Silab under the trademark Firmalift® or from LSN under the trademark Cytovitin®; or the synthesis of fibronectin, such as the extract of Salina zooplankton marketed by Seporga under the trademark GP4G®; the yeast extract available in particular from Alban Müller under the trademark Drieline®; and the palmitoyl pentapeptide marketed by Sederma under the trademark Matrixil®; or the synthesis of compounds present at the dermal-epidermal junction (such as collagen VII and/or collagen IV) and/or laminin, such as dipalmitoyl hydroxyproline, marketed in particular by Seppic under the name "Seppilift DPHP", or phytosterol sulfate, such as that marketed by Vincience under the name "Phytocohesine"; or the inhibition of metalloproteinases (matrix metalloproteinases or MMPs), such as more particularly MMP 1, 2, 3 or 9. Mention may be made of retinoids and derivatives, oligopeptides and lipopeptides, lipoamino acids, the malt extract marketed by Coletica under the trademark Collalift®; extracts of blueberry or of rosemary; lycopene; isoflavones, their derivatives or the plant extracts comprising them, in particular extracts of soybean (marketed, for example, by Ichimaru Pharcos under the trademark Flavosterone SB®), of red clover (marketed, for example, by Sederma under the trademark "Sterocare®"), of flax, of kakkon or of sage; extracts of Curcuma longa; or Siegesbeckia extracts (marketed, for example, by Sederma); or on the inhibition of serine proteases, such as leukocyte elastase or cathepsin G. Mention may be made of the peptide extract of leguminous plant (Pisum sativum) seeds marketed by LSN under the trademark Parelastyl®; heparinoids; and pseudodipeptides, such as {2-[acetyl(3-(trifluoromethyl)phenyl)amino]-3-methylbutyrylamino}acetic acid.

Other protease-inhibiting agents which can be used as compounds in the urea containing compositions are plasminogen activation inhibitors, such as, for example, tranexamic acid.

Active principles which also stimulate epidermal macromolecules, such as filaggrin and keratins, are the lupin extract marketed by Silab under the trademark Structurine®; the extract of beech Fagus sylvatica buds marketed by Gattefossé under the trademark Gatuline®; and the extract of Salina zooplankton marketed by Seporga under the trademark GP4G® and can be used as further components.

As components which stimulate the proliferation of fibroblasts and can be formulated with the composition used according to the invention are for example selected from plant proteins and polypeptides, extracts, in particular of soybean (for example, a soybean extract marketed by LSN under the name Eleseryl SH-VEG 8® or marketed by Silab under the trademark Raffermine®); and plant hormones, such as gibberellins and cytokinins.

It is also possible to use agents which stimulate the proliferation of keratinocytes and can be formulated with the compositions used according to the invention. Such agents are in particular retinoids, such as retinol and its esters, including retinyl palmitate; adenosine; phloroglucinol; the extracts of walnut meal marketed by Gattefossé; and the extracts of Solanum tuberosum marketed by Sederma.

The agents which stimulate the differentiation of keratinocytes comprise, for example, inorganic materials, such as calcium; a lupin peptide extract, such as that marketed by Silab under the trademark Structurine®; sodium β-sitosteryl sulfate, such as that marketed by Seporga under the trademark Phytocohesine®; a water-soluble maize extract, such as that marketed by Solabia under the trademark Phytovityl®; a peptide extract of Voandzeia subterranea, such as that marketed by Laboratoires Serobiologiques under the trademark Filladyn LS 9397®; and lignans, such as secoisolariciresinol.

The compositions according to the invention including one or more of the above compounds are particularly well suited for the prevention or treatment of cutaneous signs of aging, in particular of loss of firmness and/or of elasticity of the skin.

Further components preventing/treating signs of aging of the skin which might also be used as a component of the urea-containing composition of the present invention are other compounds known be increase the amount of collagen in the dermis.

An antioxidant functions, among other things, to scavenge free radicals from skin to protect the skin from environmental aggressors. Examples of antioxidants that may be used in the present compositions include compounds having phenolic hydroxy functions, such as ascorbic acid and its derivatives/esters e.g. sodium-L-ascorbate, calcium-L-ascorbate, ascorbyl palmitate; beta-carotene; catechins; curcumin; ferulic acid derivatives (e.g. ethyl ferulate, sodium ferulate); gallic acid derivatives (e.g. propyl gallate); lycopene; reductic acid; rosmarinic acid; tannic acid; tetrahydrocurcumin; tocopherol and its derivatives e.g. α-tocopherol, γ-tocopherol, δ-tocopherol; uric acid; or any mixtures thereof. Also propylgallate, octylgallate, dodecylgallate, sodium-isoascorbate, citric acid, sodium citrate, potassium citrate and tin-II-chloride. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur.

Optionally the inventively used compositions comprise vitamins. Suitable vitamins are for example vitamin A, vitamins of the vitamin B group, e.g. vitamin B1 and B5, vitamin C, vitamins of the vitamin D group such as for example vitamin D and vitamin D3, and vitamin E. As it will be obvious to the person skilled in the art, the inventively used compositions may (also) comprise suitable derivatives of said vitamins such as the vitamin D-derivative 1-fluoro-25-hydroxy-16-ene-23-yne-hexa:fluoro-cholecalciferol.

All these potential components optionally used for formulating the urea-containing compositions have to fulfil the requirement not to interfere with the effect of urea. In order to prevent and/or treat signs of aging caused by UV-exposure like sunlight or sunburn the urea-containing compositions, especially the cosmetic formulations contain as additional compounds known sunscreen and/or UV-filters.

Such sunscreens could be particulate materials, among them are zinc oxide, titanium oxide, clays and ferric chloride. Because such material might be visible and occlusive, many people consider the corresponding opaque formulations cosmetically unacceptable. Other sunscreens rely on chemicals such as p-aminobenzoic acid (PABA), oxybenzone, dioxybenzone, ethylhexyl-methoxy cinnamate, octocrylene, octyl methoxycinnamate, and butylmethoxydibenzoylmethane that are transparent or translucent on the skin. While these materials may be more acceptable cosmetically, they are still relatively short-lived and susceptible to being removed by washing or perspiration.

Therefore improved UV-filters have been developed which can be used as additional compounds like the UV-filters preventing/treating signs of aging of the skin disclosed e.g. in:
WO07025599A1 "sunscreen composition comprising a dibenzoylmethane, an aminohydroxybenzophenone, a triazine and a triazole as UV filters"; WO07002047A1 "a product release system to atomize cosmetic hair or skin compositions containing UV filters" ; WO06032741A1 "silane merocyanine sulphone derivatives; photoprotecting compositions containing same; use thereof as UV fitter";
W005094772A3 "use of benzophenone UV filters for preventing tanning";
W005094772A2 "use of UV filters for preventing tanning"; WO04082580A3"
photostabilized topical formulations of ketoprofen containing two UV filters";
WO04022015A1 "use of diketopiperazine derivatives as photostable UV-filters in cosmetic and pharmaceutical preparations"; W003039447A3 "composition containing an amino acid n-acylated ester and a polyamide-structured UV fitter";
W003039447A2 "composition containing an amino acid n-acylated ester and a polyamide-structured UV filter"; WO0239972A1 "insoluble organic UV filters and cosmetic use thereof"; WO0149686A1 "s-triazine derivatives and their use as UV filters"; W00126618A3 "composition, especially a cosmetic composition, containing a steroid and a liposoluble UV fitter"; W00126618A2 "composition, especially a cosmetic composition, containing a steroid and a liposoluble UV fitter";
WO9825922A1 "insoluble s-triazine derivatives and their use as UV filters".

The disclosed UV-filters in these publications are also incorporated by reference herein as possible components in the urea-containing compositions.

Preferably as one component in the urea-containing compositions the UV-filters is a benzotriazolylphenole or a derivative thereof, preferably a silylated derivative, capable of filtering UV-light and/or a pyrroltriazine or a derivative thereof capable of filtering UV-light and/or a heptaazaphenalene or a derivative thereof capable of filtering UV-light.

Such UV-filters are disclosed in the documents PCT/2008/003356 "Compuestos de benzotriazlilfenol sililados sustituidos" and in W02006/128791 "New derivatives of pyrrolyltriazine together with methods for obtaining them and their use as protecting agents against UV radiation" and in W02007/006807 "New derivatives of heptaazaphenalene, methods for obtaining them, and theirs use as protecting agents against UV radiation" respectively which are incorporated by reference herein.

Retinoids as components include, without limitation, retinoic acid (e.g., all-trans or 13-cis) and derivatives thereof, retinol (Vitamin A) and esters thereof, such as retinol palmitate, retinol acetate and retinol propionate, and salts thereof.

In addition to other functions/roles, retinoids have been used to retard the effects of photoaging in skin appearing to have been damaged by exposure to the sun.

For the inventive cosmetic composition marketed in Europe the respective components shall be used in amounts which are in compliance with the Council Directives 76/768/EEC and Commission Directive 95/17/EC on the approximation of the laws of the Member States relating to cosmetic products.

Wound healing is a complex process involving factors such as cells, extracellular matrix components and the cellular microenvironment. Essentially, all wound healing involves the repair or replacement of damaged tissues. The precise nature of such repair or replacement depends upon the tissues involved, although all such processes involve certain basic principles. An important aspect of wound healing is the rate at which a wound gains tensile strength against breakage.

If all skin layers are injured, new connective tissue must first fill in the wound space. Such tissue, identified as granulation tissue, is formed by deposition of extracellular matrix components such as collagen, by fibroblasts which migrate into the wound space. The synthesis and deposition of collagen is an important event in wound healing and the rate of collagen synthesis varies in different organs.

It is a further embodiment of the present invention to use a composition comprising urea for treating and/or even preventing wounds.

It could happen that large areas of the skin are affected in case of scalps and burns. Thus, synthesis of new collagen is of particular importance for the development and regeneration of skin.

Accordingly a further embodiment of the present invention is the treatment of wounds caused by burns.

Bedsores, more properly known as pressure ulcers or decubitus ulcers, are lesions caused by many factors such as: unrelieved pressure; friction; humidity; shearing forces; temperature; age; continence and medication; to any part of the body, especially portions over bony or cartilaginous areas such as sacrum, elbows, knees, ankles etc. Although completely treatable if found early, without medical attention, bedsores can become life-threatening, and indeed fatal. In patients suffering from decubitus ulcers the skin is damaged being painful and posing a risk for infections and other diseases. One of the risk factors for developing decubitus ulcers is a damaged/weak skin.

Therefore a further embodiment is the use of compositions containing urea to prevent and/or treat wounds like decubitus ulcer.

It is well known that patients suffering from diabetis have problems with wound healing also because the skin becomes dry and fissured which often leads to wounds.

A further object of the present invention is the use of urea-containing compositions to prevent and/or treat wounds of human beings suffering from diabetis.

In general, skin which is in "good condition" will in generally be more resistant to various diseases and disorders. Also, skin which not affected otherwise will in general be more likely to heal. As an intact collagen matrix is of particular importance to the overall integrity of the skin, the inventively used compositions of the present invention will be beneficial for preventing and/or treating of certain diseases and disorders.

Thus, the present invention also relates to the use of the compositions according to the invention for the treatment and/or prevention of skin diseases and/or skin disorders, in particular acne, boil, bowen's disease, ichthyosis, keratosis pilaris, porphyria, stasis dermatitis, chronic skin ulcera.

As it will be obvious to a person skilled in the art that preferably the compositions used according to the invention should not be applied/administered to patients suffering from scleroderma.

For preventing and/or treating the before mentioned disorders, diseases and/or wounds of the skin, the inventively used compositions can also comprise at least one pharmaceutically active agent.

Such further component can be at least one pharmaceutically active agent selected from the group comprising antibiotics, anti-infectives, pharmaceutically active agents against dermatoses and active agents for wound healing.

Such pharmaceutically active agents are well known in the field.

They include but are not limited to antimicrobial agents; antibacterial agents; antipruritic and antixerotic agents; antiinflammatory agents; local anesthetics and analgesics; corticosteroids; retinoids; vitamins; hormones; and antimetabolites and are at least one active agent selected from the group comprising acyclovir, amphotericins, chlorhexidine, clotrimazole, ketoconazole, econazole, miconazole, metronidazole, minocycline, nystatin, neomycin, kanamycin, phenytoin, pare-amino benzoic acid esters, octyl methoxycinnamate, octyl salicylate, oxybenzone, dioxybenzone, tocopherol, tocopheryl acetate, selenium sulfide, zinc pyrithione, diphenhydramine, pramoxine, lidocaine, procaine, erythromycin, tekacycline' clindamycin, crotamiton, hydroquinone and its monomethyl and benzyl ethers, naproxen, ibuprofen, cromolyn, retinol, retinyl palmitate, retinyl acetate, coal tar, griseofulvin, estradiol, hydrocortisone, hydrocortisone 21-acetate, hydrocortisone 17-valerate, hydrocortisone 17-butyrate, progesterone, betamethasone valerate, betamethasone dipropionate, triamcinolone -20 acetonide, fluocinonide, clobetasol propionate, minoxidil, dipyridamole, diphenylhydantoin, benzoyl peroxide, and 5-fluorouracil.

A pharmacological acceptable carrier may also be incorporated in the inventively used composition and may be any carrier conventionally used in the art.

Additional examples of pharmaceutically active agents that can be used as components in the inventively used compositions can be found e.g. in "Rote Liste - Arzneimittelverzeichnis für Deutschland" (Edition 2008, published by Rote Liste Service GmbH, Frankfurt/Main, Germany) which is incorporated by reference herein.

Additional examples of pharmaceutically active agents for use in dermatology can be found in e.g. "Hauptgruppe 32 'Dermatika' " of the Rote Liste (Edition 2008) which are incorporated by reference herein.

In particular, a pharmaceutically active agent is an agent for the treatment of any of the diseases listed before.

The person skilled in the art will be able to select the appropriate pharmaceutically active agent according to the information presented therein and his general knowledge in the field.

Depending on the specific pharmaceutically active agent, the person skilled in the art will be able to adapt the specific composition accordingly either by obvious preliminary tests or by the general knowledge in the field.

As discussed before, examples of antibiotics, anti-infectives and pharmaceutically active agents against dermatoses and for wound healing are well known in the field.

Examples of such pharmaceutically active agents can also be found e.g. in the Rote Liste which is, as outlined before, incorporated by reference herein.

Specifically, examples of antibiotics can be found e.g. in "Hauptgruppe 10 'Antibiotika/Antiinfektiva' " of the Rote Liste.

Examples of anti-infectives can be found e.g. in "Hauptgruppe 10 'Antibiotika/Antiinfektiva' as well as "Hauptgruppe 21 'Antimykotika' " of the Rote Liste.

Examples of pharmaceutically active agents against dermatoses can be found e.g. in "Hauptgruppe 32 'Dermatika' " of the Rote Liste.

Examples of pharmaceutically active agents for wound healing can be found e.g. in "Hauptgruppe 32 'Dermatika' " as well as "Hauptgruppe 85 'Wund- und Narbenbehandlungsmittel' " of the Rote Liste.

### Example

### Compositions

Emulsions were used as listed thereafter.

### Emulsion I

| COMPOUND/MIXTURE | % (weight/weight) |
|---|---|
| - Urea | 10% |
| Lipophilic emollient mixture: | 13,4% |
| - Isopropyl Myristate | |
| - Paraffinum Liquidum | |
| - Cetearyl Ethylhexanoate | |
| - Dimethicone | |
| - Persea Gratissima (Avocado) Oil | |
| Emulsifying mixture: | 7,7% |
| - Glyceryl Stearate | |
| - PEG-40 Stearate | |
| - Cetyl Alcohol | |
| - Stearic Acid | |
| - Sodium Carbomer | |
| Humectant/skin conditioning agent/pH adjuster mixture: | 4,205% |
| - Sorbitol | |
| - Allantoin | |
| - Lactic Acid | |
| Preservative mixture: | 0,27% |
| - Propylparaben | |
| - Methylparaben | |
| - 2-Bromo-2-Nitropropane-1,3-diol | |
| Fragance (Parfum): | 0,1% |
| + Water | up to 100% |

### Emulsion II

| COMPOUND/MIXTURE | % (weight/weight) |
|---|---|
| - Urea | 20% |
| Lipophilic emollient mixture: | 13,4% |
| - Isopropyl Myristate | |
| - Paraffinum Liquidum | |
| - Cetearyl Ethylhexanoate | |
| - Dimethicone | |
| - Persea Gratissima (Avocado) Oil | |
| Emulsifying mixture: | 10,3% |
| - Glyceryl Stearate | |
| - PEG-40 Stearate | |
| - Cetyl Alcohol | |
| - Stearic Acid | |
| - Sodium Carbomer | |
| - Acrylates/C10-30 Alkyl Acrylate Crosspolymer | |
| Humectant/skin conditioning agent/pH adjuster mixture: | 4,206% |
| - Sorbitol | |
| - Allantoin | |
| - Lactic Acid | |
| Preservative mixture: | 0,27% |
| - Propylparaben | |
| - Methylparaben | |
| - 2-Bromo-2-Nitropropane-1,3-diol | |
| Fragance (Parfum): | 0,1% |
| + Water | up to 100% |

### Emulsion III

| COMPOUND/MIXTURE | % (weight/weight) |
|---|---|
| - Urea | --- |
| Lipophilic emollient mixture: | 13,4% |
| - Isopropyl Myristate | |
| - Paraffinum Liquidum | |
| - Cetearyl Ethylhexanoate | |
| - Dimethicone | |
| - Persea Gratissima (Avocado) Oil | |
| Emulsifying mixture: | 9,0% |
| - Glyceryl Stearate | |
| - PEG-40 Stearate | |
| - Cetyl Alcohol | |
| - Stearic Acid | |
| - Sodium Carbomer | |
| - Acrylates/C10-30 Alkyl Acrylate Crosspolymer | |
| Humectant/skin conditioning agent/pH adjuster mixture: | 4,205% |
| - Sorbitol | |
| - Allantoin | |
| - Lactic Acid | |
| Preservative mixture: | 0,27% |
| - Propylparaben | |
| - Methylparaben | |
| - 2-Bromo-2-Nitropropane-1,3-diol | |
| Fragance (Parfum): | 0,1% |
| + Water up | to 100% |

### Study

### 1. Treatment

Tests were conducted with human volunteers. The age ranged from 21 to 59 years. All were non-smokers and had no history of any severe skin disease.
The emulsions I, II, respectively III were applied to the skin of the volunteers as following:
All volunteers were treated once daily for 4 weeks with each of the the emulsions I, II and III at three different areas of buttock skin. Skin biopsies from the three areas were taken thereafter to test the gene expression as described thereafter.

### 2. Assessment of gene expression

For assessment of gene expression total RNA was extracted from frozen biopsies (Ø 4 mm) and gene expression measured by semiquantitative RT-PCR. For isolation of RNA from frozen skin biopsies, 600 µl lysis buffer from PeqGold Total RNA Kit (PeqLab, Erlangen, Germany) were added and the samples were disrupted in a MixerMill MM300 (Retsch, Haan, Germany) three times for 3 min with 30 Hz. 50 ng total RNA were used for cDNA synthesis. PCR reactions were performed in an Opticon 1 (MJ Research, Waltham, MA, USA) using Sybr QPCR Supermix w. Rox (Invitrogen, Karlsruhe, Germany). PCR conditions were as follows: activation of hot start taq polymerase 94° C 15 minutes; denaturation 95° C, 20 seconds; annealing, 55° C, 20 seconds; extension, 72° C 30 seconds. Each sample was subjected to PCR in double using the appropriate primer pairs for 45-50 cycles. For comparison of relative expression in real time PCR control cells and treated cells the 2 ^{(-delta delta C(T))} method was used (Livak KJ, and Schmittgen TD. Analysis of relative gene expression data using real time quantitative PCR and the 2 (-delta delta C(T)) method. Methods 2001: 25: 402 - 408). mRNA expression of the genes of interest was shown as fold induction as compared to untreated skin samples of the same volunteer.
Primers used are listed in Table 1.

**Table 1: Primer pairs for real time PCR**

| Gene | Primer pairs |
|---|---|
| 18S rRNA | 5´-GCCGCTAGAGGTGAAATTCTTG-3´ 5´-CATTCTTGGCAAATGCTTTCG´-3´ |
| col1A1 | 5´-CCTGCGTGTACCCCACTCA-3´ 5´-ACCAGACATGCCTCTTGTCCTT-3´ |
| col1A2 | 5´- GATTGAGACCCTTCTTACTCCTGAA-3´ 5´-GGGTGGCTGAGTCTCAAGTCA-3´ |

For determination of relative gene expression in real time PCT the 2(-delta delta C(T))-method was used as described in Livak and Schmittgen (2001) "Analysis of relative gene expression data using real time quantitative PCR and the 2 (-delta delta C(T))-method" Methods 25: 401-408.
For statistical analysis, the experimental data were analyzed using the Wilcoxon signed-rank test.

### 3. Assessment of increase in amount of collagen

Production of collagen at the protein level can be determined using liquid chromatography/mass spectrometry. An exemplary method is described e.g. in Tredget et al. (1990) "Determination of 4-hydroxyproline in collagen by gas chromatography/mass spectrometry". Anal Biochem 1990, 190:259-265.

### 4. Results

As shown in Table 2, treatment with emulsion I as well as treatment with emulsion II resulted in a significant increase of expression of the collagen genes col1A1 and col1A2 as compared to treatment with emulsion III and as compared to the control.

**Table 2: Level of induction of expression of col1A1/col1A2 by topical application of emulsions I, II and III.**

| Gene | Emulsion | % of urea in emulsion | x-fold Induction +/- standard deviation |
|---|---|---|---|
| Col1A1 | Emulsion III | 0% | 3.7 +/- 3.4 |
| Col1A1 | Emulsion I | 10% | 6.1 +/- 9.0 |
| Col1A1 | Emulsion II | 20% | 7.3 +/- 9.4 |
| Col1A2 | Emulsion III | 0% | 4.6 +/- 5.1 |
| Col1A2 | Emulsion I | 10% | 5.6 +/- 6.6 |
| Col1A2 | Emulsion II | 20% | 6.7 +/- 8.0 |

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims

## Claims

1. A use of a composition comprising urea for increasing the expression of at least one gene associated with the collagen synthesis within the dermis of the skin.

2. A use according to claim 1 wherein said gene is col1A1 or col1A2.

3. A use according to claim 1 or 2 wherein the expression of any of said genes is increased by a factor of at least 2, preferably at least 3, more preferably at least 4.

4. A use according to any one of claims 1 to 3 wherein the amount of collagen in the treated skin is increased by at least 5%, preferably by at least 10%, more preferably by at least 20%, even more preferably by at least 50%, most preferably by at least 100%.

5. A use according to any one of claims 1 to 4 wherein said composition is applied topically onto the skin or administered by injection.

6. A use according to claim 5 wherein said composition is applied at least once a day for at least 2 weeks topically and/or administered by injection and optionally longer until the expression of any of said genes is increased by a factor of at least 2, preferably at least 3, more preferably at least 4 and/or the amount of collagen in the treated region is increased by at least 5%, preferably by at least 10%, more preferably by at least 20%, even more preferably by at least 50%, most preferably by at least 100% by weight.

7. A use according to any one of claim 1 to 6 wherein said composition comprises 1 to 30% by weight, preferable 5 to 25% by weight, based on the total amount of the composition, of urea.

8. A use according to any one of claims 1 to 7 for preventing and/or treating of damages associated with and/or facilitated by a reduced level of collagen in the skin and/or a reduced expression of genes associated with the collagen synthesis in the dermis of the skin by increasing the collagen level at least to compensate at least said reduction.

9. A use according to claim 8 wherein the treated skin is normal and or already damaged skin of an individual, preferable of a human being.

10. A use according to claim 8 or 9 for preventing and/or treating signs of aging

11. A use according to claim 10 wherein the signs of aging are wrinkles and/or loss of elasticity and or/and dischronia, preferable melasma.

12. A use according to claim 8 or 9 wherein said damages are caused by exposure to UV-radiation.

13. A use according to any one of claims 1 to 12 wherein said composition is a cosmetic composition, preferable further comprising at least one compound selected from the group comprising UV-filters, vitamins, coenzymes, retinoids, phytokines and antioxidative agents.

14. A use according to claim 13 wherein said UV-filter is at least one UV-filter selected from the group comprising pyrroltriazines, benzotriazolylphenoles and heptaazaphenalenes.

15. A use according to claim 1 to 7 for treating wounds

16. A use according to claim 15 wherein said wound is at least one burn.

17. A use according to claim 15 wherein said wound is a decubitus ulcer.

18. A use according claim 15 wherein said wounds are caused by diabetis.

19. A use according to claim 8 wherein said damages are at least one damage selected from the group comprising skin diseases and/or skin diseases, preferably acne, boil, ichthyosis, keratosis pilaris, phorphyria, stasis dermatitis, chronic skin ulcera.

20. A use according to any one of claims 1 to 12 and 15 to 19 wherein said composition is a pharmaceutical composition, preferable further comprising at least one pharmaceutically active agent.

21. A use according to claim 20 wherein said pharmaceutically active agent is selected from the group comprising antibiotics, anti-infectives, pharmaceutically active agents against dermatoses and for wound healing.
